# FASCICULE DE BREVET EUROPEEN

(11) **EP 0 549 421 B1**
(45) Date de publication et mention de la délivrance du brevet: **16.04.1997**
(21) Numéro de dépôt: 92403442.4
(22) Date de dépôt: 17.12.1992
(51) Int. Cl.: C12P 15/00, C12G 3/02

(54) **Procédé de production d'acide anthranilique et d'anthranilate de méthyle par emploi de mutants de saccharomyces cerevisiae**
Verfahren zur Herstellung von Anthranilsäure und Methylanthranilate unter Verwendung von Saccharomyces Cerevisiae Mutanten
Process for the production of anthranilic acid and methylanthranilate by using saccharomyces cerivisiae mutants

(30) Priorité: 20.12.1991 FR 9115917
(43) Date de publication de la demande: 30.06.1993
(73) Titulaire: PERNOD-RICARD, F-75008 Paris (FR)
(72) Inventeur: Vladescu Barbu-Dinu, Vladimir, F-75016 Paris (FR); Girard, Patrick, F-91940 Les Ulis (FR)
(74) Mandataire: Ahner, Francis

(56) Documents cités:
- WO-A-89/00203
- PATENT ABSTRACTS OF JAPAN vol. 14, no. 370 (C-747)(4313) 10 Août 1990
- PATENT ABSTRACTS OF JAPAN vol. 12, no. 70 (C-479)(2917) 4 Mars 1988

## Description

La présente invention concerne un procédé de production d'acide anthranilique naturel et d'anthranilate de méthyle par emploi de mutants de Saccharomyces cerevisiae et utilisation de ces mutants dans l'élaboration de boissons fermentées.

L'anthranilate de méthyle, molécule à haute valeur aromatique (note organoleptique "fleur d'oranger"), est un dérivé de l'acide anthranilique largement utilisé dans l'élaboration de certains arômes alimentaires. L'industrie des arômes emploie 27 tonnes d'anthranilate de méthyle par an (1), mais aucune source naturelle de cette molécule n'est actuellement disponible. Le seul procédé biotechnologique de production d'anthranilate de méthyle existant à ce jour consiste en une déméthylation microbiologique du N-méthylanthranilate de méthyle isolé de l'huile essentielle de mandarinier "petit grain" (2). Ce procédé est coûteux et les disponibilités d'anthranilate de méthyle ainsi préparé semblent limitées.

Par ailleurs, la biosynthèse de dérivés anthraniliques a été observée dans des cultures de Poria cocos (3) et de Pycnoporus cinnabarinus (4). Cependant, aucun procédé de production basé sur ces basidiomycètes n'a été mis au point jusqu'à présent, compte tenu des très faibles rendements obtenus, de l'ordre du milligramme par millilitre.

L'anthranilate de méthyle a été décelé également dans différents cépages de raisin de l'espèce Vitis labrusca (Niagara, Concord) ; à des concentrations de 0,5 - 3 mg/l, il serait responsable du goût foxé de certains vins américains (5).

On a découvert, de façon surprenante, selon la présente invention, que l'on pouvait obtenir un rendement très élevé de production d'acide anthranilique (jusqu'à 500 mg/ml) par la fermentation avec un mutant de Saccharomyces cerevisiae.

Plus précisément, le mutant selon l'invention est un mutant auxotrophe pour le tryptophane, bloqué dans l'étape catalysée par la 5-P-ribosylanthranilate transférase et présentant une anthranilate synthase insensible à la rétroinhibition par le tryptophane.

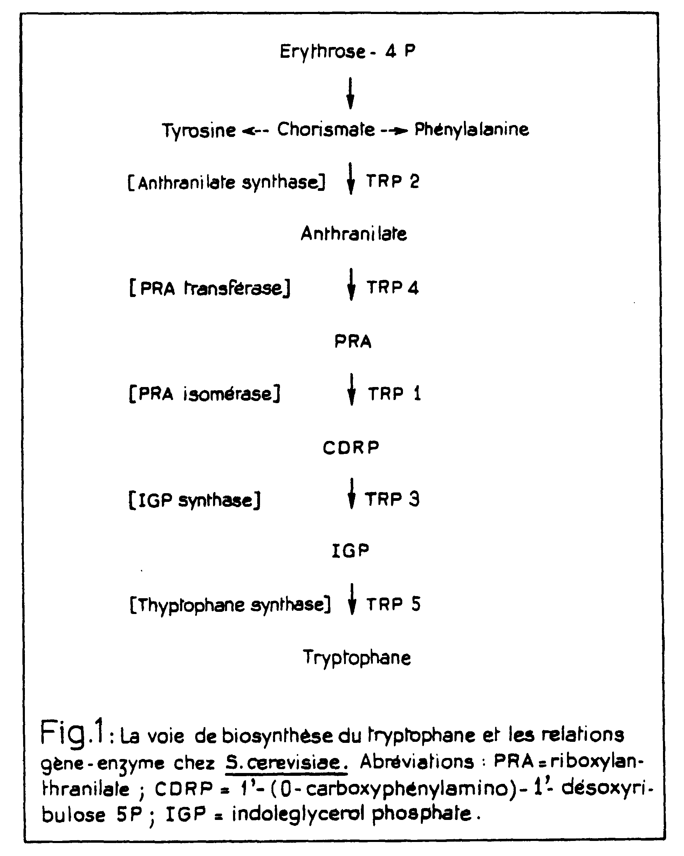

La présente invention est basée sur le fait que l'acide anthranilique est un intermédiaire de la voie de biosynthèse du tryptophane (fig. 1) ; des mutants de Saccharomyces cerevisiae bloqués dans l'étape catalysée par la 5-P-ribosylanthranilate transférase (trp 4) accumulent et excrètent de l'acide anthranilique.

Le flux métabolique sur la voie du tryptophane est régulé au niveau de l'anthranilate synthase, enzyme sensible à la rétroinhibition par le produit final de la voie, le tryptophane. Un excès de cet acide aminé entraîne l'inhibition de l'anthranilate synthase et l'acide anthranilique, ainsi que tous les autres intermédiaires de la voie, n'est plus synthétisé. Or, les mutants trp 4, étant auxotrophes pour le tryptophane, nécessitent précisément un apport de tryptophane exogène qui, à son tour, inhibe l'anthranilate synthase.

Des mutants à anthranilate synthase insensible à l'inhibition par le tryptophane ont cependant été décrits. Une souche susceptible d'accumuler de l'acide anthranilique selon l'invention doit donc porter la mutation trp 4 et, en même temps, posséder une anthranilate synthase insensible à l'inhibition par le tryptophane. Des mutants de ce type représentent une source potentielle d'acide anthranilique naturel qui peut également servir à la préparation de l'anhranilate de méthyle selon l'invention.

De préférence, la souche est une souche diploïde homozygote pour la mutation trp 4 et/ou l'insensibilisation de l'anthranilate synthase à l'inhibition par le tryptophane.

La présente invention a donc également pour objet un procédé de production d'anthranilate de méthyle par estérification de l'acide anthranilique obtenu par le procédé selon l'invention, avec du méthanol naturel d'origine agricole.

D'autre part, S. cerevisiae étant considéré comme microorganisme GRAS (generally recognized as safe), des mutants de ce type peuvent être utilisés en vinification, en vue d'une production d'anthranilate de méthyle au cours de la fermentation. Des vins ayant le goût foxé, caractéristique de certains vins américains très prisés, peuvent être ainsi obtenus, à partir de cépages autres que Niagara et Concord.

La présente invention a donc aussi pour objet une boisson fermentée riche en acide anthranilique caractérisée en ce qu'elle a été fermentée par une souche mutante de Saccharomyces cerevisiae auxotrophe pour le tryptophane, bloqué dans l'étape catalysée par la 5-P-ribosylanthranilate transférase et présentant une anthranilate synthase insensible à la rétroinhibition par le tryptophane.

En particulier, la présente invention a pour objet une boisson fermentée au goût foxé notamment à base de raisin, pomme ou malt, caractérisée en ce qu'elle a été fermentée par une souche mutante de Saccharomyces cerevisiae conforme au procédé selon la présente invention.

Les mutants selon l'invention peuvent être obtenus par tous moyens conventionnels biotechnologiques de mutagenèse et sélection de souches mutantes bien connus de l'homme de l'art en particulier.

D'autres avantages et caractéristiques de la présente invention apparaîtront à la lumière de la description détaillée d'un mode de réalisation qui va suivre.

La figure 1 représente la voie de biosynthèse du tryptophane.

La voie de biosynthèse du tryptophane et les relations gène - enzyme chez S. cerevisiae. Abréviations : PRA = 5-P-riboxylanthranilate ; CDRP = 1'-(0-carboxyphénylamino)-1'-désoxyribulose 5P ; IGP = indoleglycerol phosphate.

### 1. Construction d'une souche mutante selon l'invention

**1.1.** Plusieurs auxotrophes trp⁻ spontanés ont été isolés à partir de la souche de laboratoire FL 100 (sauvage, haploïde, de signe a). Parmi ces auxotrophes, l'un a été identifié comme trp 4 par des tests de complémentation et par analyse enzymatique (souche PR 250).
**1.2.** Parmi les descendants méiotiques d'une souche oenologique industrielle, plusieurs clônes à anthranilate synthase insensibles à l'hinhibition par le tryptophane ont été identifiés par la résistance au 5-méthyletryptophane et par la mesure in vitro de l'activité anthranilate synthase en présence de tryptophane. Le clône L116-5b a été retenu pour les étapes suivantes.
**1.3.** Le diploïde PR 250 x L116-5b a été mis à sporuler et de nombreuses asques ont été disséquées. Dans toutes les tétrades complètes, le caractère trp 4, ainsi que la résistance de l'antranilate synthase ségrègent 2 : 2 et de manière indépendante. Les clônes recombinants 9 (signe a) et 2 (signe alpha) ont servi à la construction du diploïde 2 x 9, qui a été utilisé par la suite dans toutes les expériences.
**1.4.** Dans les différentes étapes de cette procédure, les phénotypes des souches, et notamment l'excrétion d'acide anthranilique, sont révélés sur boîtes de Petri grâce à la fluorescence de l'acide anthranilique en UV.

### 2. Production d'acide anthranilique sur différents milieux

Des précultures sont effectuées en milieu YPG pendant 24 heures à 25° sous agitation (140 rpm). Les différents milieux de culture sont stérilisés à 120° pendant 20 mn (à l'exception du milieu R, qui est stérilisé par addition de 400 µl/l de diméthyle-dicarbonate) et inoculés avec 10⁶ cpm. Les cultures sont incubées 7 jours à 25° sous agitation (140 rpm) et l'acide anthranilique est dosé dans les milieux par CLHP (colonne RP 18 Merck, volume injecté 20 µl, température 25°, éluant H₂O : acétonitrile 50 : 50, détecteur UV-VISλ = 326 nm, débit 0,8 ml/mn) et par CPG après extraction au dichlorométhane (chromatographe HP 5880, colonne FFAP). La production d'acide anthranilique dans les différents milieux testés est présentée dans le tableau 1 pour le diploïde 2 x 9 en comparaison avec les souches parentales 2 et 9, étant bien entendu que les souches initiales FL 100 et la souche oenologique ne produisent de l'acide anthranilique sur aucun milieu.

Les meilleures performances sont obtenues avec le diploïde 2x9, vraisemblablement par un effet de dosage génique, l'allèle trp 4 étant représenté en dose double.

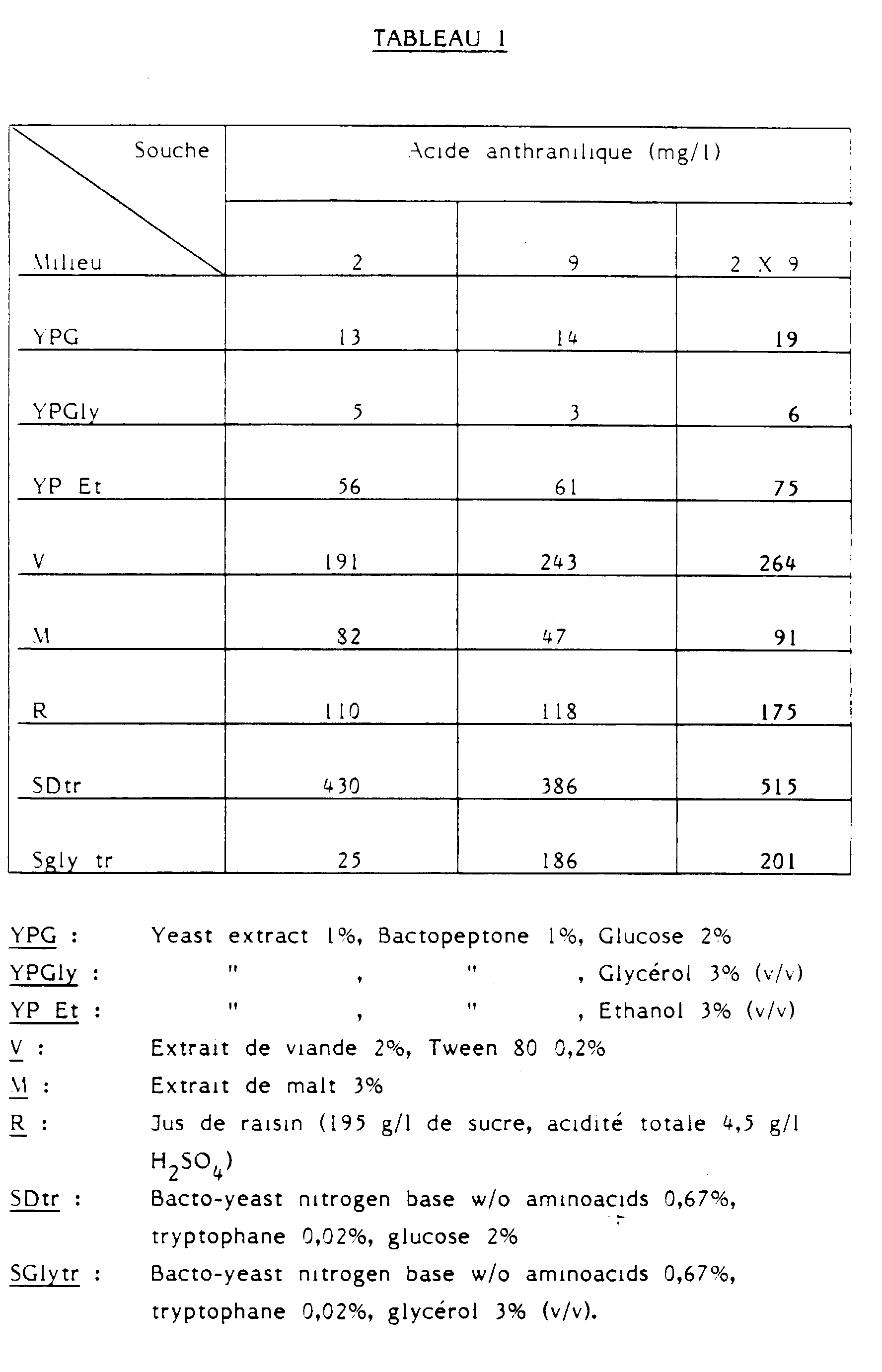

### 3. Préparation de l'acide anthranilique et de l'anthranilate de méthyle

L'acide anthranilique est préparé à partir de cultures de 7 jours du diploïde 2 X 9 en milieu SDtr, par extraction avec 1,5 volumes d'acétate d'éthyle, suivie d'une évaporation à sec. La préparation d'acide anthranilique ainsi obtenue est utilisée dans les essais d'estérification avec du méthanol : 100 mg d'acide anthranilique sont suspendus dans 20 ml de méthanol d'origine agricole. En présence de catalyseur acide, le chauffage du milieu réactionel conduit à la formation d'anthranilate de méthyle caractérisé en CPG/MS conformément aux lois d'estérification bien connues.

### 4. Fermentation du jus de raisin avec la souche 2 X 9

Du jus de raisin frais contenant 195 g/l de sucre (acidité totale 4,5 g/l H₂SO₄) est inoculé avec 10⁸ cpm et incubé en stationnaire à 20° pendant 3 semaines. En fin de fermentation, la totalité du sucre est épuisée et le taux d'alcool est d'environ 4%. Le produit fermenté présente une forte odeur d'anthranilate de méthyle, détectée sans ambiguïté par analyse sensorielle et une quantité d'acide anthranilique de 150 mg/l.

### REFERENCES

1. Welsh et al. (1989) Critical Reviews in Biotechnology 9, 105
2. Page et al. (1989) Brevet international WO 89/00203
3. Berger et al. (1987) Symp. Bioflavors Würzburg, 415
4. Gross-Falconnier (1991) Thèse Paris-Sud, Orsay
5. Miozzari et al. (1978) J. Bacteriol. 134, 48
6. Nakahara et al. (1945) Sci. Papers. Inst. Phys. Chem. Res. (Tokyo) 42, 39.

## Revendications

1. Procédé de production d'acide anthranilique naturel par fermentation, caractérisé en ce qu'on utilise pour la fermentation un mutant d'une souche de levure Saccharomyces cerevisiae auxotrophe pour le tryptophane, bloqué dans l'étape catalysée par la 5-P-ribosylanthranilate transférase et présentant une anthranilate synthase insensible à la rétroinhibition par le tryptophane.

2. Procédé selon la revendication 1, caractérisé en ce que la souche est une souche diploïde homozygote pour la mutation bloquant l'étape catalysée par la 5-P-ribosylanthranilate transférase et/ou la mutation insensibilisant l'anthranilate synthase à l'inhibition par le tryptophane.

3. Procédé de production d'anthranilate de méthyle par estérification de l'acide anthranilique obtenu par le procédé selon l'une des revendications 1 ou 2, avec du méthanol naturel d'origine agricole.

4. Procédé de préparation d'une boisson fermentée riche en acide anthranilique caractérisé en ce que ladite boisson a été fermentée par une souche mutante de Saccharomyces cerevisiae selon la revendication 1 ou 2.

5. Procédé selon la revendication 4, caractérisé en ce qu'il s'agit d'une boisson au goût foxé à base de raisin, pomme ou malt.

## Patentansprüche

1. Verfahren zur Herstellung von natürlicher Anthranilsäure durch Fermentation, dadurch gekennzeichnet, daß man für die Fermentation eine Mutante eines für Tryptophan auxotrophen Hefestammes von Saccharomyces cerevisiae verwendet, die in der durch die 5-P-Ribosylanthranilat-Transferase katalysierten Stufe blockiert ist und eine für die Retroinhibierung durch Tryptophan unempfindliche Anthranil-Synthase aufweist.

2. Verfahren nach Anspruch 1, dadurch gekennzeichnet, daß es sich bei dem Stamm um einen diploiden Stamm handelt, der homozygot ist für die Mutation, welche die durch 5-P-Ribosylanthranilat-Transferase blockierte katalysierte Stufe blockiert, und/oder für die Mutation, welche die Anthranil-Synthase für die Inhibierung durch Tryptophan unempfindlich macht.

3. Verfahren zur Herstellung von Methylanthranilat durch Veresterung der in dem Verfahren nach einem der Ansprüche 1 oder 2 erhaltenen Anthranilsäure mit natürlichem Methanol aus der Landwirtschaft.

4. Verfahren zur Herstellung eines an Anthranilsäure reichen fermentierten Getränks, dadurch gekennzeichnet, daß das genannte Getränk mit einem Mutanten-Stamm von Saccharomyces cerevisiae nach Anspruch 1 oder 2 fermentiert worden ist.

5. Verfahren nach Anspruch 4, dadurch gekennzeichnet, daß es sich dabei um ein Getränk mit Weintrauben-, Apfel- oder Malzgeschmack handelt.

## Claims

1. Process for producing natural anthranilic acid by means of fermentation, characterized in that use is made, for the fermentation, of a mutant of a Saccharomyces cerevisiae yeast strain which is auxotrophic for tryptophan, which is blocked in the step catalysed by 5-P-ribosylanthranilate transferase and which possesses an anthranilate synthase which is insensitive to feedback inhibition by tryptophan.

2. Process according to Claim 1, characterized in that the strain is a diploid strain which is homozygous for the mutation which blocks the step catalyzed by 5-P-ribosylanthranilate transferase and/or the mutation which renders the anthranilate synthase insensitive to inhibition by tryptophan.

3. Process for producing methyl anthranilate by esterifying, with natural methanol or methanol of agricultural origin, the anthranilic acid which is obtained by the process according to either of Claims 1 and 2.

4. Process for preparing a fermented beverage which is rich in anthranilic acid, characterized in that the said beverage was fermented using a mutant strain of Saccharomyces cerevisiae according to Claim 1 or 2.

5. Process according to Claim 4, characterized in that the beverage is a beverage which has a foxy flavour and which is based on grapes, apples or malt.
